# EUROPÄISCHE PATENTANMELDUNG

(11) **EP 0 601 673 A1**
(43) Veröffentlichungstag der Anmeldung: **15.06.1994**
(21) Anmeldenummer: 93250287.5
(22) Anmeldetag: 23.10.1993
(51) Int. Cl.: A61L 2/26, F28F 3/08, A61L 2/04

(54) **Kurzzeitsterilisationsmodul**

(30) Priorität: 11.12.1992 DE 4242426
(71) Anmelder: INSTITUT FÜR BIOPROZESS- UND ANALYSENMESSTECHNIK e.V., D-37308 Heiligenstadt (DE)
(72) Erfinder: Metze, Josef, Dipl.-Ing. Dr., D-37308 Heiligenstadt (DE); Hildebrandt, Egon, Dipl.-Ing., D-37308 Geisleden (DE); Gastrock, Gunther, Dipl.-Ing., D-37308 Heiligenstadt (DE); Sauerbier, Rainer, D-37308 Heiligenstadt (DE); Liefeith, Klaus, Dipl.-Ing. Dr., D-37318 Uder (DE); Hildebrand, Gerhard, Dipl.-Ing., D-37318 Lutter (DE); Kaufhold, Sebastian, D-37327 Leinefelde (DE)
(74) Vertreter: Maikowski, Michael, Dipl.-Ing. Dr.

(57) **Zusammenfassung**

Die Erfindung betrifft ein Kurzzeitsterilisationsmodul für Nährmedien, wie sie für die Fermentation benötigt werden.

Als ein Bauteil des Moduls ist für die Durchleitung des zu sterilisierenden Mediums erfindungsgemäß eine Platte angeordnet, die mindestens eine kanalartige Ausnehmung mit definiertem Querschnitt aufweist. Die Platte ist auf der Seite mit der kanalartigen Ausnehmung mit einer Heizplatte bedeckt.

## Beschreibung

Die Erfindung betrifft einen Wärmetauschermodul für die kontinuierliche Kurzzeitsterilisation, insbesondere zur Sterilisation von Nährmedien, wie sie für die Fermentation benötigt werden, und zur Dekontamination am Fermenterausgang im Havariefall.

Zur Sterilisation von Fermentationsmedien werden im Laboratoriums- und Technikumsmaßstab zumeist Batch-Sterillisationsverfahren angewendet, d.h. eine vorgegebene Menge Nährmedium wird in einem Autoklav sterilisiert, und anschließend dem Prozeß zugeführt. Ist im Verlauf der Fermentation das Medium verbraucht, muß erneut eine Vorlage mit Nährmedium sterilisiert und an den Fermenter angeschlossen werden.

Nachteilig bei dieser Verfahrensweise sind die relativ langen Aufheiz- und Abkühlzeiten.

Zur Vermeidung dieses Nachteils, ist aus der Lebensmitteltechnik bekannt, eine kontinuierliche Sterilisation durchzuführen. Dieses Verfahren ist in der Lebensmitteltechnik bereits seit langem bei der UHT-Erhitzung von Milch angewendet worden, wobei durch kurze Verweilzeiten bei hohen Temperaturen zwischen 140 - 150° C die Zersetzung des Mediums (Milch, Sahne, u. a.) weitestgehend verhindert wird.

Bedingt durch relativ große Volumenströme, die in der Größenordnung von 10³-10⁴ l/h liegen, sind bei den industriellen Sterilisationsverfahren leistungsfähige Plattenwärmeübertrager zur Aufheizung und Abkühlung eingesetzt worden. Untersuchungen an den in der Lebensmitteltechnologie eingesetzten Plattenwärmetauschern haben bei den genannten großen Volumenströmen eine gleichmäßige Durchströmung bzw. gleichmäßigen Wärmeübergang ergeben.
/Ladwig, H.P.; Reuter, H.; Strömung und örtliche Wärmeübertragung in Plattenwärmetauschern; Chem.-Ing.-Techn. 52, 1980, S. 237/
Zur weiteren Verkürzung der mit den Plattenwärmeübertragern erzielten Aufheiz- und Abkühlzeiten ist es bekannt /DE-OS 3 905 066/, bei einem Wärmetauschermodul aus gestapelten Metallfolien mit dazwischen angeordneten Abstandshaltern diese Abstandshalter als Gewebekarten mit Öffnungen auszubilden, die mit den Metallkarten deckungsgleich sind, so daß die Öffnungen bei gestapelten Karten rohrförmige Kanäle bilden.

Die weitere Verkürzung der Aufheiz- und Abkühlzeiten ist besonders bei flüssigen Medien mit thermolabilen Komponenten wichtig, z.B. mit bestimmten Zellkulturen, um deren Schädigung durch den Sterilisationsprozeß auszuschließen.

Die genannten Anordnungen haben den Nachteil, daß nur bei großen Volumenströmen die genannten positiven Effekte eintreten. Bei kleinen Volumenströmen, insbesondere zwischen 0,1 und 10 l/h ergeben sich bei diesen Anordnungen undefinierte Strömungsverhältnisse. Dadurch ist die genaue Einhaltung der Zeit, während der sich das Medium im Sterilisator befindet, nicht möglich. Das beeinflußt den Sterilisationseffekt und die Sicherheit der Sterilisation negativ, d.h. es besteht die Gefahr, daß entweder keine vollständige Sterilisation erreicht wird oder daß das Medium durch eine zu lange Verweildauer geschädigt wird.

Weiterhin besteht der Nachteil, daß wegen der undefinierten Strömungsverhältnisse eine Maßstabsvergrößerung (scale-up) der Anordnungen zur Erzielung etwas größerer Volumenströme nicht möglich ist.

Der Erfindung liegt die Aufgabe zugrunde, zur Beseitigung der genannten Nachteile einen Kurzzeitsterilisationsmodul für die kontinuierliche Sterilisation bei kleinen Volumen-strömen zu schaffen, bei dem definierte Strömungsverhältnisse für das zu sterilisierende Medium vorliegen.

Erfindungsgemäß wird das bei einem Kurzzeitsterilisationsmodul durch die Merkmale des Anspruchs 1 erreicht. Bei einem Kurzzeitsterilisationsmodul für die kontinuierliche Sterilisation, mit mindestens einem Bauteil für die Durchleitung des zu sterilisierenden Mediums und mit mindestens einem mit dem erstgenannten Bauteil im Wärmekontakt stehenden Heizkörper, ist erfindungsgemäß als Bauteil für die Durchleitung des zu sterilisierenden Mediums eine Platte angeordnet, die mindestens eine kanalartige Ausnehmung mit definiertem Querschnitt aufweist, und zur Ausgestaltung der Ausnehmung zu einem Strömungskanal ist diese von mindestens einem plattenförmiger Heizkörper bedeckt.

Vorzugsweise erstreckt sich die kanalartige Ausnehmung mäanderförmig über den plattenförmigen Heizkörper und hat der Strömungskanal einen rechteckförmigen Querschnitt.

Es ist weiterhin zweckmäßig, daß der plattenförmige Heizkörper Heizbahnen aufweist, die deckungsgleich mit dem Strömungskanal verlaufen.

Als plattenförmiger Heizkörper kann z.B. eine beheizbare Silikongummifolie oder eine dampfbeheizte Metallplatte sein.

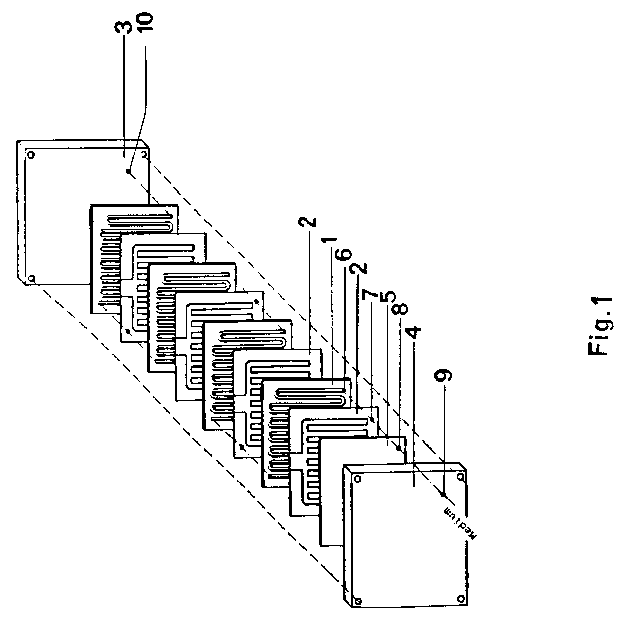

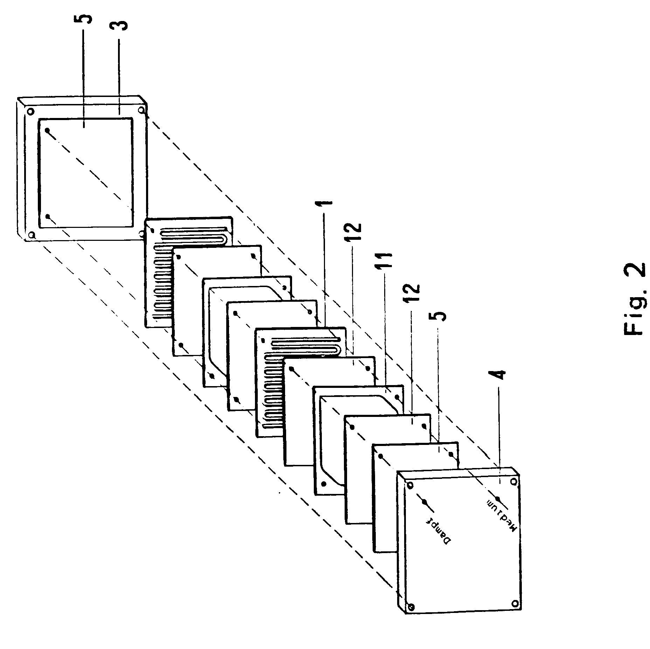

Die Erfindung soll in einem Ausführungsbeispiel anhand einer Zeichnung erläutert werden. Es zeigen:
- Fig. 1: eine Anordnung mit elektrisch beheizten Folien,
- Fig. 2: eine Anordnug mit dampfbeheizten Platten,
Die Anordnung der Fig. 1 hat Mäanderplatten 1 für das Durchströmen des zu sterilisierenden Mediums. Jede Mäanderplatte hat eine mäanderförmige Ausnehmung und ist zwischen zwei elektrisch beheizten Folien 2 angeordnet, die die mäanderförmige Ausnehmung beidseitig abdecken, so daß ein Strömungskanal gebildet wird. Die Mäanderplatten 1 und die Folien 2 sind zusammen zwischen einer Grundplatte 3 und einer Deckplatte 4 angeordnet. Zwischen der Mäanderplatte 1 und der elektrisch beheizten Folie 2 kann eine Zwischenfolie 12 (Fig. 2) angeordnet sein, um bei Verwendung einer weichen Folie 2 deren Eindringen in die mäanderförmige Ausnehmung zu verhindern. In der Fig. 1 sind die Bauteile 1 bis 4 mit Abstand zueinander dargestellt, sie bilden aber als gebrauchsfertiger Modul eine kompakte Einheit, bei der diese Bauteile über nicht dargestellte Spannbolzen dicht aufeinander gedrückt und abgedichtet werden, um einen guten Wärmekontakt zu gewährleisten.

Zur Vermeidung der Wärmeabgabe an die Grundplatte 3 und die Deckplatte 4 ist zwischen diesen und den angrenzenden Bauteilen mindestens je eine Isolierplatte 5 angeordnet.

Das zu sterilisierende Medium wird über Bohrungen 6, die in axialer Richtung mit Bohrungen 7 in den Folien 2, Bohrungen 8 in den Isolierplatten 5, einer Bohrung 9 in der Deckplatte 4 und einer Bohrung 10 in der Grundplatte 3 fluchten, zu den Mäanderplatten geleitet. Die Mäanderplatten 1 können bei dieser Anordnung seriell oder parallel vom zu sterilisierenden Medium durchflossen werden, was durch einfaches Drehen der Platten 11 (Fig. 2) und/ oder 1 erreicht wird. Die Anzahl der Mäanderplatten kann deshalb in Abhängigkeit von der benötigten Menge des Mediums verändert werden.

Bei der Anordnung der Fig.2 sind anstelle der beheizten Folien Verbundplatten 11 vorgesehen, die mit Dampf beheizt sind. Der Dampf wird wie das zu sterilisierende Medium axial dem Modul zugeführt und gelangt über entsprechende Bohrungen zu den Verbundplatten 11.

## Patentansprüche

1. Kurzzeitsterilisationsmodul für die kontinuierliche Sterilisation, mit mindestens einem Bauteil für die Durchleitung des zu sterilisierenden Mediums und mit mindestens einem mit dem erstgenannten Bauteil im Wärmekontakt stehenden Heizkörper, **dadurch gekennzeichnet,** daß als Bauteil für die Durchleitung des zu sterilisierenden Mediums eine Platte (1) angeordnet ist, die mindestens eine kanalartige Ausnehmung mit definiertem Querschnitt aufweist, und daß zur Ausgestaltung der Ausnehmung zu einem Strömungskanal diese von mindestens einem plattenförmiger Heizkörper bedeckt ist.

2. Anordnung nach Anspruch 1, **dadurch gekennzeichnet,** daß die kanalartige Ausnehmung mäanderförmig ist.

3. Anordnung nach mindestens einem der Ansprüche 1 und 2, **dadurch gekennzeichnet,** daß der Strömungskanal einen rechteckigen Querschnitt hat.

4. Anordnung nach mindestens einem der Ansprüche 1 bis 3, **dadurch gekennzeichnet,** daß der plattenförmige Heizkörper Heizbahnen aufweist, die deckungsgleich mit dem Strömungskanal verlaufen.

5. Anordnung nach mindestens einem der Ansprüche 1 bis 4, **dadurch gekennzeichnet,** daß der plattenförmige Heizkörper eine beheizbare Silikongummifolie (2) ist.

6. Anordnung nach mindestens einem der Ansprüche 1 bis 4, **dadurch gekennzeichnet,** daß der plattenförmige Heizkörper eine dampfbeheizte Metall-Verbundplatte (11) ist.
